# EUROPÄISCHE PATENTSCHRIFT

(11) **EP 0 446 178 B1**
(45) Veröffentlichungstag und Bekanntmachung des Hinweises auf die Patenterteilung: **02.11.1994**
(21) Anmeldenummer: 91810140.3
(22) Anmeldetag: 04.03.1991
(51) Int. Cl.: A61B 17/36, A61B 17/22, A61M 25/10, A61M 25/00

(54) **Angioplastie-Lichtleiterkatheter für die Stenoseabtragung mit Laserlichtenergie**
Angioplasty light guide catheter for stenosis ablation by means of laser energy
Cathéter d'angioplastie à guide de lumière pour l'ablation de sténose par énergie à laser

(30) Priorität: 05.03.1990 CH 690/90
(43) Veröffentlichungstag der Anmeldung: 11.09.1991
(73) Patentinhaber: SCHNEIDER (EUROPE) AG, 8180 Bülach (CH)
(72) Erfinder: Hofmann, Eugen, CH-8064 Zürich (CH)
(74) Vertreter: Groner, Manfred

(56) Entgegenhaltungen:
- EP-A- 0 311 458
- WO-A-87/01273
- US-A- 3 498 286
- US-A- 4 793 359

## Beschreibung

Die Erfindung betrifft einen Angioplastie-Lichtleiterkatheter nach dem Oberbegriff des unabhängigen Patentanspruchs 1.

Der Einsatz von Laserstrahlen zur Behandlung von Stenosen ist schon vor mehr als 25 Jahren vorgeschlagen worden. Obwohl auch dieses Verfahren neben der bekannten Ballondilatation eine Behandlung ohne chirurgischen Eingriff ermöglicht und von allem Anfang an vielversprechend war, ist dennoch die Oeffnung stenosierter Gefässe mit Laserlicht kaum über das experimentelle Stadium gediehen. Ein wesentlicher Grund wird darin gesehen, dass kein geeigneter Katheter zur Verfügung stand, der eine genaue Positionierung der Lichtleiter im Gefäss ermöglichte. Ebenfalls ist bisher die Gefahr einer Dissektion der Gefässwandung nicht gelöst.

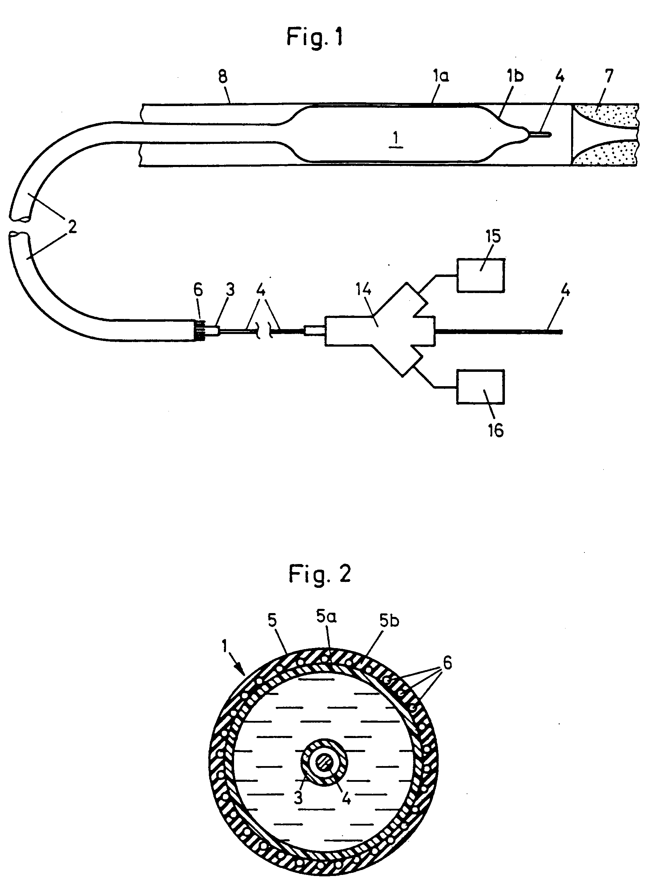

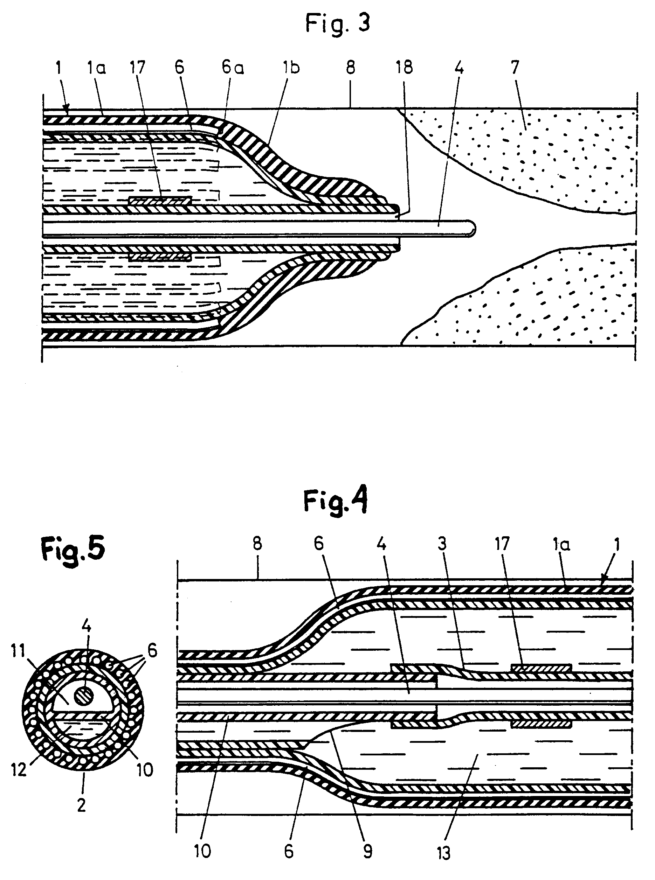

Bekannt ist ein Angioplastie-Lichtleiterkatheter, bei dem das Laserlicht mit Linsen gebündelt wird. Weiter ist aus einem Artikel der Zeitschrift Herz+Gefäss 5 (1985) Seite 185, Abbildung 5, ein Angioplastie-Lichtleiterkatheter bekannt, bei welchem drei konzentrisch angeordnete Laserlichtleiter durch einen Ballon wandständig im Gefäss gehalten werden können. Auch bei diesem Katheter ist eine genaue Positionierung und Kontrolle des Laserlichtes nicht möglich und ist die Gefahr einer Dissektion vergleichsweise hoch. Zudem besteht hier der Nachteil, dass mit einem Katheter mit wandständigem Laser Lichtleiter hochgradige Verschlüsse nicht erfolgreich behandelt werden können. Bei solchen hochgradigen Verschlüssen wäre ein Katheter mit einem zentral angeordneten Lichtleiter geeignet, wie er aus der Zeitschrift JACC Band 8 (5), November 1986; 1989-95 (Fig. 1), oder aus US-A-4 793 359 bekannt ist, besser geeignet.

Der Katheter gemäß WO-A-87/01273 weist exzentrisch angeordnete Lichtleiter auf, deren Enden mittels aufblasbarem Ballon so gebogen werden, daß die austretende Laserstrahlung auf zentral liegende Stenosen gerichtet wird. Durch zusätzliches Drehen des Katheters kann das gesamte stenosierte Lumen bestrahlt werden.

Der Erfindung liegt die Aufgabe zugrunde, einen Katheter der genannten Gattung zu schaffen, mit dem das Laserlicht sicher und einfach kontrollierbar ist und der ohne Gefahr einer Dissektion der Gefässwand zu Behandlung von Stenosen unterschiedlicher Grade geeignet ist.

Diese Aufgabe wird durch die Erfindung gemäss Anspruch 1 gelöst. Beim erfindungsgemässen Katheter ist der Abstand der Austrittsstellen der Lichtleiter von der Mittellinie des Ballons eine Funktion des Druckes der Ballonflüssigkeit. Dieser Druck kann vom Chirurgen genau und in einfacher Weise verändert und kontrolliert werden. Bei kleinem Druck und entsprechend kleinem Durchmesser des Dilatationsballons ist das Laserlicht in der Gefässmitte konzentriert und eignet sich dadurch besonders zur Behandlung hochgradiger Stenosen. Bei höherem Druck liegen die Austrittsstellen entsprechend näher an der Gefässinnenwand und können entsprechend auch die aussenliegenden Bereiche von Stenosen abgetragen werden. Weitere vorteilhafte Merkmale ergeben sich aus den abhängigen Ansprüchen und der Beschreibung.

Ein Ausführungsbeispiel der Erfindung wird nachfolgend anhand der Zeichnungen näher erläutert. Es zeigen:
- Fig. 1: schematisch ein stenosierter Gefässabschnitt mit eingesetztem Dilatationsballon,
- Fig. 2: ein Querschnitt durch einen Dilatationsballon,
- Fig. 3: ein Längsschnitt durch das distale Ende eines dilatierten Ballons in einem schematisch dargestellten stenosierten Gefässabschnitt,
- Fig. 4: ein Längsschnitt durch den hinteren Bereich eines Dilatationsballons, und
- Fig. 5: ein Querschnitt durch den Schaft eines erfindungsgemässen Katheters.

Die Fig. 1 zeigt rein schematisch ein Blutgefäss 8 mit einer Stenose 7 und einen in das Gefäss perkutan eingesetzten Katheter mit einem Dilatationsballon 1 und einem Katheterschaft 2. Der Katheter wird mit einem Führungsdraht 4 in das Gefäss 8 eingesetzt. Der Führungsdraht 4 erstreckt sich durch den Schaft 2 und den Dilatationsballon 1 und kann an einer distalen Oeffnung 18 austreten. Wie die Fig. 5 zeigt, ist der Schaft 2 durch eine Wand 10 in ein Lumen 11 für den Führungsdraht 4 und ein Lumen 12 für Druckflüssigkeit unterteilt. Das Lumen 12 ist gemäss Fig. 4 über eine Oeffnung 9 mit dem Balloninnern 13 und am proximalen Ende mit einer Saug- und Druckpumpe 16 verbunden. Ueber die Pumpe 16 kann der Druck im Innern 13 des Dilatationsballons 1 genau reguliert und damit der Durchmesser des Ballons reguliert werden. Bei der Einführung des Katheters ist der Dilatationsballon 1 in bekannter Weise gefaltet. Im entfalteten Zustand ist der Dilatationsballon 1 wie aus Fig. 1 ersichtlich zwischen den beiden sich verjüngenden Enden aussenseitig zylindrisch.

Die Wandung 5 des Dilatationsballons 1 besteht gemäss Fig. 2 aus einer inneren Schicht 5a und einer äusseren Schicht 5b. Die innere Schicht 5a besteht aus einem vergleichsweise weichen Kunststoff und die äussere Schicht 5b vorzugsweise aus Silikonkautschuk. Die Wandung 5 des Dilatationsballons 1 ist im Gegensatz zu den bekannten Dilatationsballonen elastisch in gewissen Grenzen dehnbar. Auf die innere Schicht 5a sind Lichtleiterfasern oder -bündel 6 aufgeklebt und von der äusseren Schicht 5b überdeckt. Diese Lichtleiter 6 sind gleichmässig über den Umfang der Wandung 5 verteilt und verlaufen parallel zur Längsrichtung des Katheters. Die Austrittsstellen 6a der Lichtleiter 6 liegen bei dilatiertem Ballon 1 auf einem Kreis und in einer Ebene, welche den Dilatationsballon etwa im Uebergang zwischen seinem zylindrischen Bereich 1a und seinem distalen Verjüngungsbereich 1b schneidet. Die Lichtleiter 6 erstecken sich auch über die ganze Länge des Schaftes 2 und sind über ein Anschlussstück 14 mit einem Lasergerät 15 gekoppelt. Das Gerät 15 ist vorzugsweise ein pulsierender Xenon-Chlorid Excimer-Laser. Das vom Lasergerät 15 erzeugte Licht gelangt über das Anschlussstück 14 und den Schaft 2 bis zu den Austrittsstellen 6a der Lichtleiter 6.

Die Lichtleiter 6 weisen einen Durchmesser von beispielsweise 0,1 mm auf und sind sehr flexibel. Der Dilatationsballon 1 kann somit auch mit den eingelegten Lichtleitern 6 gefaltet werden. Der radiale Abstand der Lichtleiter 6 von der Mittellinie des Dilatationsballons 1 wächst mit zunehmendem Druck der im Ballon vorhandenen Flüssigkeit. Entsprechend ist der Abstand der Austrittsstellen 6a von der Mittellinie des Dilatationsballons 1 eine Funktion des über die Pumpe 16 regulierbaren Druckes. Da die Wandung 5 elastisch dehnbar ist, ist es über die Druckregulierung möglich, die Austrittsstellen 6a in Kreisen unterschiedlicher Radien anzuordnen. Entsprechend treffen die an den Austrittsstellen 6a austretenden Laserstrahlen mehr oder weniger weit von der Gefässinnenseite entfernt auf die Stenose 7. Bei vergleichsweise kleinem Druck im Dilatationsballon ist somit ein entsprechend intensives Laserlichtbündel auf das Zentrum der Stenose gerichtet. Bei höherem Druck werden hingegen die eher äusseren Bereiche der Stenose 7 mit weniger dichtem Laserlicht abgetragen.

Der Dilatationsballon 1 wird zur Abtragung der Stenose 7 mit Laserlicht wie in Fig. 3 gezeigt vor der Stenose 7 positioniert. Mit einem Markierstreifen 17 kann die Position des Dilatationskatheters 1 beobachtet werden. Neben der Abtragung der Stenose mit Laserlichtenergie ist mit dem erfindungsgemässen Katheter auch die Anwendung der üblichen Ballondilatation denkbar. Ohne Katheterwechsel kann somit vor oder nach einer Behandlung mit Laserlichtenergie eine Stenose 7 mit dem Dilatationsballon 1 gedehnt werden.

## Patentansprüche

1. Angioplastie-Lichtleiterkatheter für die Stenoseabtragung mit Laserlichtenergie, mit einem perkutan in das zu behandelnde Gefäss einzuführenden Dilatationsballon (1) und mehreren Lichtleitern (6), die im Bereich des Dilatationsballons (1) zur Auskuppelung und am proximalen Ende eines Schaftes (2) des Katheters zur Einkuppelung des Laserlichtes enden, dadurch gekennzeichnet, dass die Lichtleiter (6) wenigstens im Bereich des Dilatationsballons (1) einen Durchmesser aufweisen, der nicht wesentlich grösser ist als die Stärke der Wandung (5) des Dilatationsballons (1) und, dass die Lichtleiter derart mit der Wandung (5) verbunden sind, dass sie an ihren distalen Enden (6a) durch Druckänderungen im Dilatationsballon (1) radial nach aussen und nach innen bewegbar sind.

2. Katheter nach Anspruch 1, dadurch gekennzeichnet, dass die Lichtleiter (6) flexible Fasern mit einem Durchmesser kleiner als etwa 0,5 mm sind.

3. Katheter nach Anspruch 1 oder 2, dadurch gekennzeichnet, dass die Lichtleiter (6) in die Wandung (5) des Dilatationsballons (1) eingebettet sind.

4. Katheter nach einem der Ansprüche 1 bis 3, dadurch gekennzeichnet, dass die Wandung (5) eine innere Schicht (5a) aufweist, auf welcher eine äussere Schicht (5b) aufgetragen ist, und dass die Lichtleiter (6) in der äusseren Schicht (5b) eingebettet sind.

5. Katheter nach Anspruch 4, dadurch gekennzeichnet, dass die äussere Schicht (5b) aus Silikonkautschuk hergestellt ist.

6. Katheter nach einem der Ansprüche 1 bis 5, dadurch gekennzeichnet, dass die Wandung (5) des Dilatationsballons (1) aus einem vergleichsweise weichen und flexiblen Kunststoffmaterial hergestellt ist.

7. Katheter nach einem der Ansprüche 1 bis 6, dadurch gekennzeichnet, dass wesentlich mehr als 3 Lichtleiter (6) und vorzugsweise mehr als 20 Lichtleiter (6) vorgesehen sind.

8. Katheter nach einem der Ansprüche 1 bis 7, dadurch gekennzeichnet, dass er an einem pulsierenden Xenon-Chlorid Excimer-Laser angeschlossen ist.

## Claims

1. A light guide catheter employed in angioplasty for stenotic abrasion using laser light energy, comprising a dilating balloon (1) to be introduced percutaneously into the vessel to be treated, and a plurality of light guides (6) which end within the region of the dilating balloon (1) in order to disconnect the laser light and end at the proximal end of a shaft (2) of the catheter in order to connect the laser light, characterised in that the light guides (6), at least within the region of a dilating balloon (1), have a diameter which is not substantially larger than the thickness of the wall (5) of the dilating balloon (1), and that the light guides are connected to the wall (5) in such a way that, as a result of pressure changes in the dilating balloon (1), they are radially movable both outwardly and inwardly at their distal ends (6a).

2. A catheter according to claim 1, characterised in that the light guides (6) are flexible fibres with a diameter of less than about 0.5 mm.

3. A catheter according to claim 1 or 2, characterised in that the light guides (6) are embedded into the wall (5) of the dilating balloon (1).

4. A catheter according to any one of claims 1 to 3, characterised in that the wall (5) has an inner layer (5a) to which an outer layer (5b) is applied, and that the light guides (6) are embedded in the outer layer (5b).

5. A catheter according to claim 4, characterised in that the outer layer (5b) is made of silicone rubber.

6. A catheter according to any one of claims 1 to 5, characterised in that the wall (5) of the dilating balloon (1) is made of a comparatively soft and flexible plastics material.

7. A catheter according to any one of claims 1 to 6, characterised in that more than three light guides (6), and preferably more than twenty light guides (6), are provided.

8. A catheter according to any one of claims 1 to 7, characterised in that it is connected to a pulsating xenon-chloride excimer laser.

## Revendications

1. Cathéter d'angioplastie à guide de lumière pour l'ablation de sténoses par énergie à lumière laser comprenant un ballon de dilatation (1) qui est introduit par voie cutanée dans le vaisseau à traiter et qui comprend plusieurs guides de lumière (6) qui se terminent dans la région du ballon de dilatation (1) en vue du découplage et à l'extrémité proximale de la tige (2) du cathéter en vue du couplage de la lumière laser, caractérisé en ce que les guides de lumière (6) ont un diamètre, au moins dans la zone du ballon de dilatation (1), qui n'est pas sensiblement supérieur à l'épaisseur de paroi (5) du ballon de dilatation (1), et en ce que les guides de lumière sont reliés à la paroi (5) de manière qu'ils puissent être déplacés radialement vers l'extérieur et vers l'intérieur à leurs extrémités distales (6a) par des modifications de la pression dans le ballon de dilatation (1).

2. Cathéter selon la revendication 1, caractérisé en ce que les guides de lumière sont des fibres flexibles d'un diamètre inférieur à environ 0,5 mm.

3. Cathéter selon la revendication 1 ou 2, caractérisé en ce que les guides de lumière (6) sont noyés dans la paroi (5) du ballon de dilatation (1).

4. Cathéter selon l'une quelconque des revendications 1 à 3, caractérisé en ce que la paroi (5) comprend une couche interne (5a) sur laquelle est appliquée une couche externe (5b), et en ce que les guides de lumière (6) sont noyés dans la couche externe (5b).

5. Cathéter selon la revendication 4, caractérisé en ce que la couche externe (5b) est réalisée en caoutchouc silicone.

6. Cathéter selon l'une quelconque des revendications 1 à 5, caractérisé en ce que la paroi (5) du ballon de dilatation (1) est réalisée en une matière synthétique relativement molle et flexible.

7. Cathéter selon l'une quelconque des revendications 1 à 6, caractérisé en ce que l'on prévoit sensiblement plus de trois guides de lumière (6) et de préférence plus de 20 guides de lumière (6).

8. Cathéter selon l'une quelconque des revendications 1 à 7, caractérisé en ce qu'il est raccordé à un laser excimer pulsant à chlorure de xénon.
